# EUROPEAN PATENT APPLICATION

(11) **EP 4 119 968 A1**
(43) Date of publication of application: **18.01.2023**
(21) Application number: 21305986.8
(22) Date of filing: 15.07.2021
(51) Int. Cl.: G01R 33/48, A61B 5/00, G01N 33/00

(54) **A METHOD FOR MEASURING THE RESPONSE TO OLFACTIVE STIMULI**

(71) Applicant: Takasago International Corporation, Tokyo 144-8721 (JP)
(72) Inventor: WARR, Jonathan, PARIS (FR)
(74) Representative: Cabinet Beau de Loménie

(57) **Abstract**

The invention relates to a method of screening a fragrance or a flavouring using functional Magnetic Resonance Imaging to assess the ability of said fragrance or flavouring to elicit a "well-being" effect.

## Description

### Technical Field of the Invention

The invention relates to the use of a quantitative functional Magnetic Resonance Imaging (fMRI), mapping technique to discern human responses to stimuli in particular stimuli arising from fragrance ingredients, accords, fully formulated fragrances, or flavourings, which induce a well-being effect.

### Background of the Invention

Traditional consumer research techniques have only limited success in predicting whether a product will be a commercial success or not. It is desirable to have a technique of measuring brain response to stimuli without the time and reflection required in formulating and expressing ideas verbally, in response to questions. Magnetic resonance imaging techniques offer a way of investigating how a consumer responds to a stimulus in a non-verbal way while the brain interprets the stimulus; it allows visualization of both primary response (e.g. in the piriform cortex, PC) and further processing of the sensory signals (e.g. in the orbitofrontal cortex, OFC). The consumer has to use or experience the product in order to react to it and in the context of measuring brain response by MRI this may be difficult for many products. However, it is possible to introduce olfactive stimuli to subjects in an accurate and precise way, whilst measuring brain response, using a specific form of olfactometer.

An olfactometer is a device designed to provide a number of olfactive stimuli to subjects in a controlled and reproducible way. The requirements of the MRI scanning technique impose restrictions on the design of olfactometers suitable for use in conjunction with MRI scanners. One major restriction is the elimination of magnetic materials from within the vicinity of the scanner. Due to the availability of olfactometers which can be used in conjunction with MRI scanners the interest in measuring brain response by MRI to olfactive stimuli has increased significantly in the past few years. Most of the interest in MRI measurement with olfaction has centered on hedonic responses to stimuli (see for example Zatorre R.L. Jones-Gottman M, Rouby C, Neural mechanisms involved in odor pleasantness and intensity judgements Neuroreport 11 2711-2716 (2000*)* or Kobal G, Kettenmann B, Int. J. Psychophysiology 36 (2) 157-163 2000*).*

The Angular Gyrus (AG), part of the temporal-parietal junction (TPJ), has attracted attention because of its association with the integration of signals from other brain areas, and in particular its link to social context (see for example Seghier, Neuroscientist 2013, v19 p43 or Carter and Huttel, Trends Cogn. Sci. 2013, v17 p32).

Studies used to draw such conclusions used diverse stimuli, for example words (Thakral et al., The Journal of Neuroscience, 2017, v37 p8142), texts (Uddin et al., Cerebral Cortex, 2010, v20 p2636)*,* or images (Apperly et al., Cognition. 2007, v103 p300)*.*

Other brain areas that have been associated with the interpretation of social cues include the Frontal Inferior Cortex, which includes the Frontal Inferior Gyrus, and the Frontal Medial Cortex, which includes the Frontal Medial Gyrus; see for example Henco et al., Cortex, 2020 v131, p221-236 or Mainieri et al., NeuroImage, 2013, v81, p294-305*.*

The notion of "well-being" has become increasing widely used, and many people spontaneously consider they can judge their state of "well-being". Whilst no single definition exists, for the purposes of the present disclosure we refer to the interpretation provided by the Health Related Quality of Life (HRQOL) program in Centre for Disease Control and prevention's (CDC's) Division of Population Health, which provides expertise and support in population HRQOL and well-being assessment to CDC, states, communities, and other public health partners. In summary they state (https://www.cdc.gov/hrgol/wellbeing.htm - accessed 2021/11/04):
- There is no consensus around a single definition of well-being, but there is general agreement that at minimum, well-being includes the presence of positive emotions and moods (e.g., contentment, happiness), the absence of negative emotions (e.g., depression, anxiety), satisfaction with life, fulfilment and positive functioning. In simple terms, well-being can be described as judging life positively and feeling good.
- Well-being is a positive outcome that is meaningful for people and for many sectors of society, because it tells us that people perceive that their lives are going well. Good living conditions (e.g., housing, employment) are fundamental to well-being. Tracking these conditions is important for public policy. However, many indicators that measure living conditions fail to measure what people think and feel about their lives, such as the quality of their relationships, their positive emotions and resilience, the realization of their potential, or their overall satisfaction with life - i.e., their "well-being". Well-being generally includes global judgments of life satisfaction and feelings ranging from depression to joy.
- Well-being integrates mental health (mind) and physical health (body) resulting in more holistic approaches to disease prevention and health promotion.

Surprisingly we have found that fragrances and flavourings that have been identified by respondents as having a high "well-being" association, when compared to those with a lower "well-being" association, show activation of the Angular Gyrus (AG), and optionally also show activation of the Frontal Inferior Cortex and/or the Frontal Medial Cortex. We interpret this activation as being linked to the complex and rich associations of the respondents with these fragrances or flavourings. In the present disclosure no distinction is made if the right or left hemisphere is activated, or both.

### Summary of the Invention

In one aspect, the present invention provides a method of identifying a fragrance or a flavouring which elicits a "well-being" effect, wherein the method comprises determining whether the Angular Gyrus is activated in subjects smelling said fragrance or flavouring. In some embodiments the method further comprises determining whether the Frontal Inferior Cortex and/or the Frontal Medial Gyrus Cortex is/are also activated in said subjects.

In another aspect, the present invention provides a fragrance or a flavouring identified by the above-mentioned method.

In another aspect, the present invention provides a method of preparing a fully formulated fragrance or an accord, which comprises identifying a fragrance which elicits a "well-being" effect by the above-mentioned method, and formulating said fragrance into a fully formulated fragrance or an accord. The present invention also provides a method of preparing a flavouring preparation, a food product, an oral care product or a beverage which comprises identifying a flavouring which elicits a "well-being" effect by the above-mentioned method, and formulating said flavouring into a flavouring preparation, a food product, an oral care product or a beverage.

In another aspect, the present invention provides the use of a fragrance which elicits a "well-being" effect as identified by the above-mentioned method in a consumer product. The invention also provides the use of a flavouring which elicits a "well-being" effect as identified by the above-mentioned method in a flavouring preparation, a food product, an oral care product or a beverage.

### Definitions

As used herein, the term "brain activity" means physiological and biochemical activity within the human brain, or a region of the brain, associated with mental activity, including but not limited to, increases in blood flow to active brain regions, changes in oxygen level in the blood, increases in metabolic activity (e.g., glucose consumption), changes in electrical potential of neurons, and the release of neurotransmitters. Brain activity can be measured non-invasively by, for example, measuring changes in electrical fields, magnetic fields emanating from the cranium, or by the blood oxygen level dependant (BOLD) method, which is a recognised technique for measuring brain activity which correlates with the increased energy consumption by the brain and the contrast between oxyhaemoglobin and de-oxyhaemoglobin.

As used herein, the term "brain area" refers to a volume of tissue within the human brain, which can be of any shape and which can be characterized anatomically or spatially.

As used herein, the terms "frontal", "anterior", "posterior", "superior" and "inferior" have their customary meanings in anatomy. See, for example, Stedman's Medical Dictionary.

Specific locations within the brain, or volumes within the brain, can also be described by reference to three-dimensional coordinate systems. One such system is that described by Talairach and Tournox (Stereotaxic Coplanar Atlas of the Human Brain, published by Thieme in 1988, ISBN 9783137117018) and is based upon a single brain considered by the authors to be typical. The brain images or maps of individual subjects can be compared to such template brains by visual comparison, or computer software programs can be used which map the individual brains onto a template brain. For example, the Statistical Parametric Mapping (SPM) software, described below, automatically performs spatial registration and normalization of individual brains onto the MNI (Montreal Neurological Institute) template. Software is also available which determines the correspondence amongst MNI coordinates and Talairach coordinates (e.g., *MRIcro, available at www.cla.sc. edu*/*psyc*/*faculty*/*rorden*/*mricro.html; see also* Rorden and Brett (2000), Behavioural Neurology, 12:191-200*).*

As used herein, the term "voxel" refers to a multidimensional data point corresponding to a specific volume in space, and particularly refers to such a data point obtained from a brain imaging procedure and corresponding to a specific volume within the brain. Voxel size is dependent on the experimental procedure and equipment, notably the resolution of the fMRI instrument. In this application a 3.0 Tesla instrument was used but instruments up to 7 Tesla are available commercially today. It should be noted that are two types of "voxel" referred to in the present disclosure in relation to the fMRI experiment, the voxel from the functional sequence, and the voxel from the anatomical sequence. Unless explicitly stated, all mentions of voxel refer to the voxel from the functional sequence.

As used herein, the term "brain activation map" means a set or array of data in which each data point corresponds to a point or volume in a human brain. Each data point can consist of a single datum associated with a brain coordinate, or can consist of a multidimensional data array associated with a brain coordinate. The brain activation map can be displayed as a two-or three-dimensional representation, or can be stored as a data set without being graphically displayed.

As used herein, the term "fragrance", is taken to mean:
- any individual material, e.g. a "fragrance ingredient" (which is synonymous with the terms "perfume ingredient" and "perfume material");
- a mixture of individual materials as defined above, such as for example an accord or a fully formulated fragrance. A mixture of individual materials may comprise at least 5, at least 10, at least 20, at least 30, or at least 40 fragrance ingredients. An accord typically has less than 25 individual materials and has a singular or simple scent direction e.g. apple accord, musky accord. A fully formulated fragrance can be made from combining accords, or one or more accords and individual ingredients, or multiple individual ingredients and will typically have 50-75 individual materials, and a more complex scent description e.g. fruity-floral-woody.

The skilled person will appreciate that a fragrance ingredient may itself comprise many individual chemical compounds and possess a pleasant smell. This distinction is understood by those familiar with the art of fragrance creation. A perfume ingredient or perfume material can be any natural oil or extract, or chemical compound used in a fragrance composition. Natural oils and extracts are described in The Essential Oils by E Guenther published by Van Nostrand and may include extracts and distillates from any part of suitable plants: roots, rhizomes, bulbs, corms, stem, bark, heartwood, leaves, flowers, seeds and fruit. Examples of such extracts and distillates include citrus fruit oils such as orange or lemon oil, tree oils such as pine oil or cedarwood oil, herb oils such as peppermint oil, thyme oil, rosemary oil, clove oil or flower extracts such as rose oil, or geranium oil. A wide variety of synthetic odiferous materials are also known for perfumery use, including materials possessing a variety of chemical functional groups, such as acetals, alkenes, alcohols, aldehydes, amides, amines, esters, ethers, imines, nitriles, ketals, ketones, oximes, thiols, thioketones, etc. Without wishing to be limited, in most cases, perfume ingredients are odiferous compounds having molecular weights between 70 mass units and 400 mass units to ensure sufficient volatility. Fragrance ingredients will not contain strongly ionizing functional groups such as sulphonates, sulphates, or quaternary ammonium ions.

A "flavouring" is taken to mean any individual material, or mixture of a few up to more than 40 individual materials, taken in the mouth, perceived principally by the senses of taste and smell, and also the general pain and tactile receptors in the mouth, as received and interpreted by the brain. A flavouring can lead to a singular or simple flavour direction e.g. apple flavour and meat flavour, or a more complex flavour direction e.g. apple pie and BBQ flavour. The perception of a flavour is a property of flavourings.

"Flavourings" are products that are added to food to impart, modify, or enhance the flavour of food (see the Guidelines for the use of Flavourings, CAC/GL 66-2008, a document prepared by the Joint FAO/WHO Expert Committee on Food Additives (JECFA)). Flavourings may consist of flavouring substances (which include natural flavouring substances and extracts as well as synthetic flavouring substances), natural flavouring complexes, thermal process flavourings or smoke flavourings and mixtures thereof and may further contain non-flavouring food ingredients. Flavourings may consist of a few up to more than 40 components listed above. Flavourings are not intended to be consumed as such, and can be incorporated into flavouring preparations, food products, oral care products or beverages. Flavourings may also contain additives (e.g. solvents or solubilisers such as surfactants) that are compatible with the products into which they are incorporated.

More extended explanation of some of the above terms can be found in the above-mentioned Guidelines, in Regulation (EC) No 1334/2008 on flavourings, and in Regulation (EC) 1333/2008 on food additives. An additional useful reference is the International Organization of the Flavor Industry (IOFI) Code of Practice, Revision April 2020 (https://cdn2-assets-servd.host/erratic-warthoci/production/Documents/iofi-code-of-practice-5th-revision.pdf accessed 2021/07/15) which explains in more detail the regulatory context and Ingredients for flavouring. It should be noted that Oral Care products are considered Cosmetic Products and not food products, and the flavours used in Oral Care products need to satisfy both The International Fragrance Association (IFRA) standards, and the requirements for a flavouring of a food product (see IFRA, Guidance for the use of IFRA standards, May 4 2020, 1.6.2, https://ifrafragrance.org/docs/default-source/ifra-code-of-practice-and-standards/49th-amendment/ifra-49th-amendment-(att-01)---guidance-for-the-use-of-ifra-standardsa7006c445f36499bbb0eb141e8c0d4be.pdf?sfvrsn=7fb244c8 2 accessed 2021/07/15).

The perception of flavour from a food product, an oral care product or a beverage involves the combination of gustatory and olfactory stimuli, giving rise to descriptors such as "fruity",' "meaty",' "floral",' "herbal"," etc. There is a distinction between orthonasal smell when sniffing (that gives the aroma of food, the bouquet of the wine) and retronasal smell when air is pulsed out from the back of the nose during the swallowing action. It is the retronasal aromas or scents that are combined with gustatory cues that gives rise to flavours (Spence, Cell 2015, 161, 24-35). Odour perception is therefore key to both the appreciation of fragrances and flavours.

Perfume and flavouring ingredients are described more fully in S. Arctander, Perfume Flavors and Chemicals. Vols. I and II, Montclair, N.J., the Merck Index, 8th Edition, Merck & Co., Inc. Rahway, N.J*.,* Allured's Flavor and Fragrance Materials 2013 Published by Allured Publishing Corp ASIN: B01FKWD33S*, and* Surberg and Panten Eds, Common Fragrance and Flavor Materials : Preparation, Properties & Uses, 6th Edition, Published by Wiley-VCH (2016) ISBN-10 : 3527331603*,* all of which are incorporated herein by reference.

As used herein, the expression "oral care product" is intended to mean a product which cleanses the oral cavity, freshens the breath, and/or maintains good oral hygiene. Examples of oral care products include toothpastes, mouthwashes and breath fresheners.

### Brief description of the figure

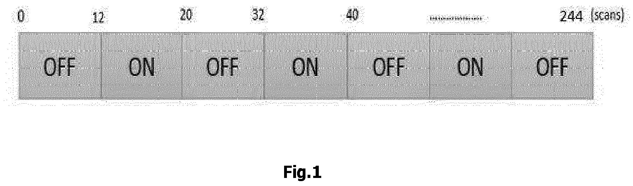

Figure 1 represents a fMRI experimental block design used in the method of the invention.

### Detailed Description of the Invention

In one aspect, the present invention provides a method of identifying a fragrance or a flavouring which elicits a well-being effect, wherein the method comprises determining whether the Angular Gyrus is activated in subjects smelling said fragrance or said flavouring.

In some embodiments the method further comprises determining whether the Frontal Inferior Cortex and/or the Frontal Medial Gyrus Cortex is/are also activated in said subjects.

In some embodiments the method comprises:
a) submitting a group of subjects to a first protocol comprising:
   - having each subject of said group smell a control odour;
   - capturing functional Magnetic Resonance Imaging (fMRI) brain scans of each subject smelling the control odour so as to detect the brain activity of each subject;
b) submitting the same group of subjects to a second protocol comprising;
   - having each subject of said group smell a fragrance or a flavouring substance to be tested;
   - capturing fMRI brain scans of each subject smelling the fragrance or the flavouring to be tested so as to detect the brain activity of each subject;
c) averaging the brain activity of all subjects as obtained in the first protocol and in the second protocol; and
d) contrasting the resulting averaged brain activity obtained in the second protocol with the resulting averaged brain activity obtained in the first protocol and determining from the contrast a number of adjacent activated voxels in the Angular Gyrus;
wherein if the number of adjacent activated voxels thus determined (from the contrast) is equal to or greater than a threshold value, then the tested fragrance or the tested flavouring elicits a well-being effect.

In some embodiments, step d) further comprises determining from the contrast a number of adjacent activated voxels in the Frontal Inferior Cortex (FIC) and/or the Frontal Medial Cortex (FMC), wherein additional confirmation that the tested fragrance or the tested flavouring elicits a well-being effect is obtained if the number of adjacent activated voxels determined (from the contrast) in the FIC and/or the FMC is equal to or greater than the same threshold value as used for the Angular Gyrus.

Sniffing any odour elicits brain activity due to odour processing, which is detected by fMRI as detailed below. The present invention is based on the surprising finding that activation of the Angular Gyrus and optionally of the Frontal Inferior Cortex and/or the Frontal Medial Cortex, in response to smelling a fragrance or a flavouring is evidence that the odour is associated with a state of "well-being".

The method of the invention comprises a first step, step a), where subjects within a group of subjects each smell a control odour and the brain activity of each subject is determined using fMRI. The group of subjects typically comprises at least 5 subjects, preferably at least 10 subjects. The control odour is preferably air or a solvent diluted in air. Any odourless solvent commonly used in fragrance or flavouring creation, e.g. di-propylene glycol, propylene glycol, MCT (Medium Chain Triglycerides) oil or triethyl citrate, can be used in the method of the invention. In some embodiments, the control odour can also be a fragrance or a flavouring as defined above (e.g. an individual material such as, for example coffee extract, or an accord or an existing fragrance or flavouring against which a new fragrance or flavouring is tested) which has been shown (using fMRI) not to activate the Angular Gyrus.

In a second step, step b), the same group of subjects smells a test fragrance or a test flavouring and the brain activity of each subject is again determined using fMRI.

The control odour and the fragrance or the flavouring are delivered to one of the subjects' nostrils via an olfactometer. To avoid the nasal passages becoming dry, the control odour and the fragrance or flavouring are preferably humidified before being introduced into the nose.

In a third step, step c), the brain activity of all subjects having smelt the control odour is averaged, and the brain activity of all subjects having smelt the test fragrance or flavouring is also averaged.

In a fourth step, step d), the brain activities averaged in step c) for the test fragrance or flavouring and the control odour are contrasted. As a result of the contrast the number of adjacent activated voxels in 3D space, i.e. adjacent voxels with significantly different blood flow (or activity), namely voxels which pass student t-test (p<0.005 or more preferably p<0.001) in the brain area studied is determined. Voxels are typically cubes, or cuboids, measuring, for example, about 0.5 mm to about 7 mm per side. When voxels are adjacent to one another they typically form what is known as a cluster. The number of voxels in a cluster is referred to as the cluster size. To determine if a brain area has been activated, the number of adjacent activated voxels obtained from the contrast is compared to a threshold value. A threshold value of 10 is commonly used in fMRI research, for example see Liebermann and Cunningham, Soc Cogn Affect Neuroscience, 2009 Dec 4(4), p423*.*

Accordingly, in some embodiments the threshold value is 10. In some embodiments, the threshold value is 20. In some embodiments, the threshold value is 30. In some embodiments, the threshold value is 50.

If the number of adjacent activated voxels from the contrast of the test fragrance or the test flavouring with the control odour is equal to or greater than the threshold value, in the Angular Gyrus, then the tested fragrance or flavouring elicits a well-being effect. Additional confirmation that a test fragrance or flavouring elicits a well-being effect can come from performing the same analysis to the Frontal Inferior Cortex and the Frontal Medial Cortex.

In some embodiments, steps c) and d) are respectively replaced by steps c1) and d1):
c1) contrasting, for each subject, the brain activity obtained in the second protocol with the brain activity obtained in the first protocol whereby a number of adjacent activated voxels is determined;
d1) averaging the resulting contrasted brain activity of all subjects.

A typical example of the overall experimental approach used is described below.

### Subject Selection

It is required that subjects be able to read and write and have the capacity to provide informed consent. Potential subjects are excluded if they have current or past psychiatric disorder other than simple phobias but including substance abuse/dependence as determined by the Structured Clinical Interview for DSM-IV Axis I Disorders (SCID-I) (First et al, Structured clinical interview for DSM-IV axis I disorders - patient edition. SCID-I/P, Version 2.0. New York, Biometric Research Department: New York State Psychiatric Institute (1995)); a history of neurologic disease; a currently unstable medical condition; used psychotropic medication within 5 half-lives of the procedure time; any metal implants or shrapnel which would make an MRI procedure unsafe; irremovable medical devices such as pacemakers or fixed hearing aids; previous inability to tolerate an MRI procedure; or claustrophobia severe enough to induce substantial anxiety in closed spaces. Other exclusion criteria include age under 9 years, history of any disease known to have an impact on the olfactory function (e.g. Diabetes, Parkinson Disease, Renal failure, etc). A complete ENT (ear, nose and throat) examination excluded pathology that could interfere with the olfactory ability: acute or severe chronic rhinitis or sinusitis, severe septum deviation, history of trauma, nasal polyps, etc. Subjects also completed a handedness inventory for the participation in the experiment. It is preferred to avoid variations due to gender or handedness, so subjects are selected to comprise panels or groups of a single sex and having the same predominant hand.

Having passed the selection criteria above only normosmic subjects were selected for the test. Various tests are available commercially to ensure that subjects have a normal sense of smell. Such tests vary from odor identification tests to more sophisticated threshold and discrimination tests. Any suitable test should be validated and reliable. In the example given below a "Sniffin' Sticks" test was used to assess the olfactory function. For further information on Sniffin Sticks see T Hummel et al. Sniffin Sticks: Olfactory Performance Assessed by the Combined Testing of Odor Identification, Odor Discrimination and Olfactory Threshold in Chem. Senses 1997 vol 22 pp39-52 or T Hummel et al. Screening Olfactory Function with a Four Minute Odor Investigation Test Reliability Normative Data and Investigations in Patients with Olfactory Loss in Ann. Otol. Rhinol. and Laryngol. 2001 vol 110 pp976-981*.* Sniffin Sticks are available from Burghardt Gmbh Wedel Germany. For odour presentation the cap is removed by the experimenter for approximately 3s and the pen's tip is placed approximately 2cm in front of both nostrils. The odour identification test involves the assessment of 12 common odours (cinnamon, banana, lemon, liquorice, pineapple, coffee, cloves, rose, leather, fish, orange, peppermint). Using a multiple choice task, identification of individual odorants was performed from a list of 4 descriptors per odour. The interval between odour presentations was 20-30s. All measurements were performed in a quiet, air-conditioned room. 10 or more correct responses were required for participation in the subsequent experiments.

### Preparation of test samples

Test fragrances and test flavourings were prepared to have approximately equal olfactive intensity using an expert panel.

### fMRI Scanner

Any suitable MRI device can be used to achieve the desired fMRI images, which is capable of operating using a spin echo, echo planar imaging (SE-EPI) sequence. This EPI protocol is optimized detecting subtle changes in blood oxygen levels in the brain over time. EPI scanning is an effective way to measure changes in the blood oxygen level dependent (BOLD) signal which has been shown to correlate reliably with changes in neural activity. Scans were taken which covered the whole brain allowing the continuous monitoring of the whole brain throughout each assessment. Suitable MRI scanners are available from Siemens AG, Phillips, GE Healthcare, Varian, Toshiba and Hitachi.

### Olfactometer

Any olfactometer can be used, which is suitable for use with an fMRI scanner. Suitable olfactometer designs have been reported by Kobal (Electroencephalography and clinical neurophysiology 71, 241-250, 1988), Sobel (J. Neuroscience methods 78, 115-123, 1997*)* and Sommer et al (J. Neurosci. Methods 209, 189-194, 2012*).* Suitable commercial olfactometers are available from Burghart Medezintechnik GmbH of Wedel Germany. To minimize head movement odorants are applied intranasally by means of a canula with an inner diameter of 2-3 mm. This canula is inserted ∼1 cm into the naris such that its opening lies beyond the nasal valve. Presentation of odourants does not simultaneously activate mechano- or thermoreceptors in the nasal mucosa, as odour pulses are embedded in a constantly flowing thermostatted (36°C), humidified (80% RH) air stream (typically 1-8 l/min) which quickly becomes undetectable after a few minutes. Hence, subjects do not perceive any change when the olfactometer switches from a no-stimulus to a stimulus condition and vice versa, nor do subjects experience any interference from mechanical or thermal stimulation. Air flow rates are determined by mass-flow controllers, which along with switching valves, are computer-controlled. Thus, the equipment allows the setup of sequences of stimuli with different quality, intensity, duration, or interstimulus interval and multiple repetitions to achieve the maximum accuracy and precision in sample presentation.

### Statistical data Analysis

Methods for the statistical analysis of changes in brain activity are well known in the art and, for some brain activity measuring devices, computer software packages are commercially available which are specifically adapted to analyze the data. For example, SPECT, PET or MRI data can be analyzed using the Dot or EMMA (Extensible MATLAB^{®} Medical image Analysis) packages which are both freely available from the MNI, or the Statistical Parametric Mapping (SPM) software package which is freely available from the Functional Imaging Laboratory of the Wellcome Department of Imaging Neuroscience at the University College of London, UK (www.fil.ion.ucl.ac.uk/spm/). The EMMA and SPM software are based upon the MATLAB^{®} programming language (MathWorks, Inc., Natick, Mass.), with additional routines in the C programming language. An SPM module is incorporated into the commercially available MEDx software (Medical Numerics, Inc., Sterling, Va. USA).

The SPM software uses a parametric statistical model at each voxel, using a general linear model to describe the variability of the data in terms of experimental and confounding effects, and residual variability. Hypotheses expressed in terms of the model parameters are assessed at each voxel with univariate statistics. Temporal convolution of the general linear model for fMRI enables the application of results from serially correlated regression, permitting the construction of statistic images from fMRI time series. The multiple comparisons problem of simultaneously assessing all the voxel statistics is addressed using the theory of continuous random fields, assuming the statistic image to be a good lattice representation of an underlying continuous stationary random field. Results for the Euler characteristic lead to corrected p-values for each voxel hypothesis. In addition, the theory permits the computation of corrected p-values for clusters of k voxels exceeding a given threshold, and for entire sets of supra-threshold clusters, leading to more powerful statistical tests at the expense of some localizing power (see Friston et al., Magnetic Resonance in Medicine 35346-3551996 and citations thereof).

The statistical approach used to evaluate the general set of fMRI data is a time-series variant of the Analysis of Variance (ANOVA) form of a general linear model. The statistical analysis tests each voxel of the brain, for each subject, against the null hypothesis (that over the duration of the testing the rise and fall of the BOLD signal coming from that voxel does not correlate with the onsets and offsets of the cycles of presentation of odours). A weighted model is created that begins with a simple square wave type model of the on-off timing events for a single task variable of interest.

The ANOVA model allows regressors to be used to model nuisance variables. In the present invention, the global signal strength of the whole brain is used as such a regressor. The global signal strength of the brain will account for fluctuations in the brain caused by respiratory cycles, cardiac flow cycles, blinking etc. These signal variations occur across the whole brain and may often be a greater magnitude than the localized changes in the BOLD signal resulting from the olfactory signal. These signal variations occurring across the brain can be subtracted from the measured fMRI signals to show the signals resulting from the odour stimuli.

The resulting product of the ANOVA computation on a single-person's fMRI data is a 3-dimensional matrix of t-values which can be represented as a 3-dimensional map. This t-value map can then be converted into a probability map (a map of corresponding p-values) and the results can be displayed graphically at whatever threshold is desired (e.g. p<0.005). The results may be overlain on a higher-resolution MRI image, in order to facilitate identification of finer-grained cortical structures.

In order to combine the data from more than one subject, each subject's brain is first normalized into a common 3-dimensional stereotactic space before each individual's t-map is computed. Then the value of the sum of the contrast weights for each voxel from each subject computed during the ANOVA (basically, the numerator of the t-statistic) is entered as a single data point in a new, "second-level" t-statistic computation. In this second-level computation, then, the mean value for each voxel across subjects is modeled as the effect term and the variance between subjects as the error term. An important consequence of this approach to keep in mind is that it is very unlikely that a voxel will show significant activation on the group-level map, unless virtually all of the subjects show activation at that voxel. Also, brain areas are only considered to be activated if a number of adjoining voxels (from the contrast) show a statistical significance above the designated probability standard.

For purposes of statistical analysis and graphical display, the raw data on brain activity is usually grouped into voxels corresponding to fixed volumes of the subject brain. The voxel size can be varied depending upon the resolution capability of the brain activity measuring device or the desired degree of precision in identifying brain regions. It should be noted, however, that smaller voxels have worse signal to noise ratios and greater susceptibility artifacts due to partial volume effects. Functional voxels are cubes, or cuboids measuring, for example, 0.5 mm to 7 mm per side (e.g., 2.4×2.4×3.0 mm³). The data can then be displayed graphically by colour-coding the voxels according to some statistical value and showing cross-sections in which levels of activity or changes in levels of activity are mapped in two-dimensions. By generating a series of such co-planar cross-sections, the entire brain volume can be mapped.

When conducting statistical analyses on brain images, investigators can select an appropriate probability value for assessing statistical significance. The particular value chosen can vary depending upon the purpose of the statistical analysis and the level of certainty required. In the studies described in the example the level for statistical significance was chosen to be p<0.001.

The method described above makes it possible to identify fragrances or flavourings which elicit a well-being effect through stimulation of the Angular Gyrus, and optionally stimulation of the Frontal Inferior Cortex and/or the Frontal Medial Cortex.

Accordingly, in another aspect the present invention relates to fragrances and flavourings identified by said method.

Such fragrances may be accords or fully formulated fragrances, or may be used to formulate accords or fully formulated fragrances that will in turn elicit a well-being effect through the activation of the angular gyrus, and optionally activation of the Frontal Inferior Cortex and/or the Frontal Medial Cortex, when smelled or worn.

Another aspect of the invention thus relates to a method for preparing an accord or a fully formulated fragrance which comprises:
a) identifying at least one fragrance which elicits a well-being effect by the method described above;
b) formulating said at least one fragrance sample thus identified into an accord or a fully formulated fragrance.

Fragrances identified to elicit a well-being effect by the method described above can be used in consumer products such as household products, candles, laundry products, personal care products and cosmetic products (including alcoholic fragrances and eau de cologne). Such products include detergents, e.g. laundry detergents, fabric softeners and conditioners, air care, toilet care, shampoos, hair conditioners, skin lotions, body oils, deodorants, sunscreen products.

In some embodiments the fragrance comprises one or more of orange oil, citrus oil, ethyl vanillin and nonalactone gamma.

Another aspect of the invention relates to a method of manufacturing a consumer product as defined above, which method comprises:
a) identifying at least one fragrance which elicits a well-being effect by the method described above;
b) incorporating said at least one fragrance thus identified into a consumer product.

In some embodiments the at least one fragrance identified is formulated into an accord or a fully formulated fragrance before incorporation into the consumer product.

Flavourings identified to elicit a well-being effect by the method described above can be used in flavouring preparations, food products, oral care products or beverages.

Another aspect of the invention thus relates to a method for preparing a flavouring preparation, a food product, an oral care product or a beverage which comprises:
a) identifying at least one flavouring which elicits a well-being effect by the method described above;
b) formulating said at least one flavouring thus identified into a flavouring preparation, a food product an oral care product or a beverage.

In some embodiments the flavouring comprises one or more of orange oil, citrus oil, ethyl vanillin and nonalactone gamma.

The invention is illustrated by the following, non-limiting example.

### Example

44 subjects were recruited (23 male and 21 female) for this study with a mean age of 25 years old. Data for 1 subject was removed from the fMRI analysis due to excessive movement. Subjects received a structured medical history (Welge-Luessen and Hummel editors, 2013, Management of Smell and Taste Disorders - A Practical Guide for Clinicians, published by Thieme, ISBN 9783131545213, p49-57, DOI: 10.1055/b-002-89586) which included questions on demographics, smoking and drinking habits, medications, current disorders, family history of any neurodegenerative disease and general nasal health status.

All subjects reported a normal sense of smell which was ascertained using an odour identification test (with maximum score of 16) from the "Sniffin' Sticks" olfactory test battery (Oleszkiewicz et al., 2018, The Laryngoscope, 128(7), 1518-1522, hhttps.//doi.org10.1002/lary.26985)*.* This test was performed within a forced choice paradigm where subjects have to identify 16 odours at supra- threshold concentrations using flash cards with four descriptors each (Kobal et al., 2000, European Archives of Oto-Rhino-Laryngology: Official Journal of the European Federation of Oto-Rhino-Laryngological Societies (EUFOS): Affiliated with the German Society for Oto-Rhino-Laryngology - Head and Neck Surgery, 257(4), 205-211. https://doi.org/10.1007/s004050050223)*.* Participants reached an average score of 13.6 ± 1.4 (mean ±SD).

The experiments were conducted according to the Helsinki declaration. The ethics committee at the medical faculty of the Technical University of Dresden approved the study design. Subjects were recruited by putting flyers on campus. They provided written informed consent.

Prior to the fMRI experiment the respondents were invited to smell a series of odours (neat oil from bottles with an opening of 6 cm in diameter) which were presented in a randomised order and asked to judge their association with "well-being". The results are shown in the Table below (visual analogue scale: left hand end [score 0] - "not associated with well-being at all"; right hand end [score +10] - "highly associated with well-being"), and hedonic ratings were also made on a visual analogue scale: left hand end [score -5] - "extremely unpleasant"; right hand end [score +5] - "extremely pleasant"):

**Table 1**

| N= 44 | Hedonic (Mean ± SD) | Well-being (Mean ± SD) |
|---|---|---|
| White flower | 3.23 ± 1.75 | 7.45 ± 1.90 |
| Citrus | 2.77 ± 1.68 | 7.05 ± 1.66 |
| Coffee | -0.43 ± 2.48 | 4.98 ± 2.17 |

Odour White flower - Fragrance formula IMP234647C - was created around a vanilla effect (ethyl vanillin) and a coconut effect (nonalactone gamma).

Odour Citrus - Fragrance/Flavouring formula FJJ232836D - was created around cold-pressed orange oil, fractionated orange oil, so called folded orange oil, and other cold-pressed citrus oils.

White flower and citrus odours were selected as having a strong association with well-being.

Odour Coffee - Coffee C'less CI-1020 - is a natural coffee extract purchased from Robertet SA, France) suitable for use in fragrance and flavourings. It was selected as not having a strong association with well-being.

As stated previously, samples were prepared to have an approximately equal odour intensity, as judged by an expert panel.

Following the above test to select suitable odours, the respondents or subjects were invited to participate in the fMRI experiment.

Odour stimuli were delivered bilaterally to both nostrils of subjects in the scanner via an air dilution computer controlled olfactometer (*Sommer, J. U., W. Maboshe, M. Griebe, C*

Heiser, K Hormann, B. A. Stuck, and T. Hummel. "A Mobile Olfactometer for Fmri-Studies." J Neurosci Methods 209, no. 1 (Jul 30 2012): 189-94. https://dx.doi.Org/10.1016/j.jneumeth.2012.05.026)*,* that allowed alternation between olfactory (ON) and non olfactory (OFF) stimulation conditions. Odours were embedded in a 2L/min constant airflow. Each functional run was comprised of 11 OFF or "baseline" blocks (14 scans each) and 10 ON blocks (9 scans each). Odours were delivered intranasally during the ON session for 8 seconds and odourless air for 12 seconds during the OFF session. Each subject underwent four functional runs with one type of odour presented in one run. The odours were randomly presented to the subjects lying in the scanner. Across subjects the sequence of these runs was randomized using a Latin Square. Subjects could communicate orally through the scanner intercom system as needed. After the functional runs, anatomy scans were acquired. Between runs 2 min breaks were included. The overall scanning time was approximately 50 min. The results for 3 stimuli are reported.

Participants were trained to use the velopharyngeal closure technique (breathing only through the mouth by lifting the soft palate) during the scan. This technique enables olfactory stimulation to be unaffected by respiration patterns. This procedure also prevents the participants from sniffing of the odours which produces percept-unrelated activation in olfactory-eloquent structures (Mainland & Sobel, 2006, Chem. Senses 31, p 181-196). Figure 1 shows the fMRI experimental block design showing paradigm of odour presentation where ON session = 9 scans (10 blocks) and OFF session = 14 scans (11 blocks) totalling 244 scans.

Data acquisition performed on a 3T MRI scanner (Siemens Medical Systems, Erlangen, Germany) at the Neuroimaging Center, Germany using a 32- channel head coil. A total of 248 functional images were collected per individual using a T2 single-shot echo-planar imaging (EPI) sequence: TR = 869 ms, TE = 38 ms, 58° flip angle, no interslice gap, 210 X 210 mm field of view. A high-resolution structural T1 image was acquired using a 3D magnetization prepared gradient rapid acquisition gradient echo (MPRAGE) sequence (TR = 2000 ms, TE = 1.95 ms, 256×256 mm² field of view, (anatomical) voxel size 1×1×1 mm³ from the anatomical sequence).

Task-driven general linear model approach (GLM) using Statistical Parametric Mapping (SPM) software (Penny et al.,2006, Statistical parametric mapping: The analysis of functional brain images. Elsevier. ISBN: 9780123725608) was used for analysis of the fMRI data. Neuro-imaging data were pre- and post-processed using SPM12 (Wellcome Department of Cognitive Neurology, London, UK, implemented in Matlab R2018b; The MathWorks, Inc., Natick, MA, USA). Functional image volumes were pre-processed, starting with realignment and unwarp. The voxel size from the functional sequence was 2.4x2.4x2.4 mm³. Functional data were registered, realigned to correct the problems of movement and then coregistered to the corresponding structural images. Further the spatially normalized (stereotactically transformed into MNI ICBM152-space; MNI-template supplied by SPM12) and smoothed images (by means of a 8x8x8 mm3 FWHM Gaussian filter) were analyzed.

From the main block design (Figure 1), 10 blocks of ON and OFF phases were used to increase stimulus related BOLD signals. We compared ON phases for presented stimuli, versus OFF phases (air), and ON_{well-being} versus ON_{not well-being} for N= 43. To restate and add further detail, each functional run comprised of alternate ON and OFF periods. In total, there were 10 ON periods, each of 8 seconds and 11 OFF periods each of 12 seconds, and each period consisted of several scans. For the analysis, we discarded the last OFF block and used 10 OFF and 10 ON blocks. SCANS: For OFF, we chose the last 9 scans from 14 (12/0.869 - 12 time periods of 0.869 seconds) scans and for ON we chose the last 5 scans from 9 (8/0.869) scans per period (or phase or block). As there is delay in BOLD we chose the last scans from ON and OFF with TR (Temporal Resolution) of 0.869 seconds. In a simplified way we can say:
ON AVERAGE based on 4 runs x 10 blocks x 5 scans (8 samples of 0.869s),
OFF AVERAGE based on 4 runs x 10 blocks x 9 scans (12 samples of 0.869s).

Whole brain activations (from the contrasts) significant at Family Wise Error (FEW) corrected <0.05 and p uncorrected < 0.001 with cluster size (k) ≥ 10 voxels are reported. For clusters having multiple peaks, one with the highest t- value was chosen. MNI coordinates are presented at x, y and z, L- left hemisphere, R- right hemisphere. Significant brain regions were labelled with AAL3 toolbox - Automated Analytical Labelling Atlas - (http://www.gin.cnrs.fr/en/tools/aal-aal2/) (Rolls et al., 2020, NeuroImage, 206, 116189, https://doi.Org/10.1016/j.neuroimage.2019.116189)*.*

To restate, a cluster, k, of a minimum 10 adjacent activated voxels in 3D space was set as the threshold value (p uncorrected <0.001), for the experiment based on the SPM analysis when contrasting the fragrance test sample to the control odour. "YES" means at least 10 adjacent voxels were activated. The contrast was first determined for a well-being associated odour (fragrance test sample) versus air as the control odour (considered to be odourless) and then for a well-being associated odour (fragrance test sample) versus a non well-being associated odour as the control odour. Results are presented in Table 2 (ON vs OFF) and Table 3 (ON_{well-being} vs ON_{not well-being}) below.

**Table 2**

| **Odour (ON> OFF)** | **K** | **k≥10 YES / NO** | **x y z** | **Region** |
|---|---|---|---|---|
| COFFEE | - | NO | - | - |
| | | | | |
| CITRUS | 61 | YES | -44 50 2 | Frontal Medial Cortex_L |
| | 13 | YES | -50 40 -4 | Frontal Inferior Cortex_L |
| | 24 | YES | 44 50 6 | Frontal Medial Cortex_R |
| | 19 | YES | -44 44 16 | Frontal Medial Cortex_R |
| | 203 | YES | -44 -62 44 | Angular Gyrus_L |
| | 30 | YES | 36 -68 50 | Angular_Gyrus R |
| | | | | |
| WHITE FLOWER | 247 | YES | 46 -64 48 | Angular_Gyrus R |
| | 229 | YES | -38 -70 50 | Angular_Gyrus L |
| | 73 | YES | -48 18 -4 | Frontal Inferior Cortex_L |
| | 40 | YES | -42 44 0 | Frontal Medial Cortex_L |
| | 15 | YES | -22 46 28 | Frontal Medial Cortex_L |

The results show that the odours Citrus and White Flower activated the Angular Gyrus, the Frontal Inferior Cortex and the Frontal Medial Cortex, whereas the Coffee odour activated none of these brain areas.

From the result in Table 2, consistent with Table 1 we can assign Citrus and White Flower odours as being associated with well-being, and the Coffee odour as not being associated with well-being

**Table 3**

| | **k** | **k≥10 YES/NO** | **x y z** | **Region** |
|---|---|---|---|---|
| Citrus vs Coffee (Citrus > Coffee) | 41 | YES | 58 -52 24 | Angular gyrus R |
| | | | | |
| White flower vs Coffee (White flower > Coffee) | 42 | YES | 56 -50 26 | Angular gyrus R |

| | | | | |
|---|---|---|---|---|
| ON_{well-being} corresponds to Citrus or White Flower ON_{not well-being} corresponds to Coffee | | | | |

The results show that the odours Citrus and White Flower activated the Angular Gyrus when compared to Coffee odour.

In conclusion, both odours associated with well-being activated the Angular Gyrus.

## Claims

1. A method of identifying a fragrance or a flavouring which elicits a well-being effect, wherein the method comprises determining whether the Angular Gyrus is activated in subjects smelling said fragrance or said flavouring.

2. The method of claim 1, which further comprises determining whether the Frontal Inferior Cortex and/or the Frontal Medial Cortex is/are activated in subjects smelling said fragrance or said flavouring.

3. The method of claim 1, comprising:
a) submitting a group of subjects to a first protocol comprising:
- having each subject of said group smell a control odour;
- capturing functional Magnetic Resonance Imaging (fMRI) brain scans of each subject smelling the control odour so as to detect the brain activity of each subject;
b) submitting the same group of subjects to a second protocol comprising:
- having each subject of said group smell a fragrance or a flavouring to be tested;
- capturing fMRI brain scans of each subject smelling the fragrance or the flavouring to be tested so as to detect the brain activity of each subject;
c) averaging the brain activity of all subjects as obtained in the first protocol and in the second protocol; and
d) contrasting the resulting averaged brain activity obtained in the second protocol with the resulting averaged brain activity obtained in the first protocol and determining from the contrast a number of adjacent activated voxels in the Angular Gyrus;
wherein if the number of adjacent activated voxels thus determined is equal to or greater than a threshold value, then the tested fragrance or the tested flavouring elicits a well-being effect.

4. The method of claim 3, wherein step d) further comprises determining from the contrast a number of adjacent activated voxels in the Frontal Inferior Cortex and/or the Frontal Medial Cortex, wherein additional confirmation that the tested fragrance or the tested flavouring elicits a well-being effect is obtained if the number of adjacent activated voxels thus determined is equal to or greater than said threshold value.

5. The method of claim 3 or claim 4, wherein steps c) and d) are respectively replaced by steps c1) and d1):
c1) contrasting, for each subject, the brain activity obtained in the second protocol with the brain activity obtained in the first protocol whereby a number of adjacent activated voxels is determined;
d1) averaging the resulting contrasted brain activity of all subjects.

6. The method of any one of claims 3-5, wherein the control odour is air or an odourless perfumery solvent diluted in air.

7. The method of any one of claims 3-6, wherein the group of subjects comprises at least 5, preferably at least 10 subjects.

8. The method of any one of claims 3 to 7, wherein the threshold number of adjacent activated voxels is ten.

9. A method for preparing an accord or a fully formulated fragrance which comprises:
a) identifying at least one fragrance which elicits a well-being effect by the method of any one of claims 1-8;
b) formulating said at least one fragrance thus identified into an accord or a fully formulated fragrance.

10. Use of a fragrance which elicits a well-being effect in a household product, a laundry product, a candle, a personal care product or a cosmetic product.

11. The use of claim 10, wherein the fragrance comprises one or more of orange oil, citrus oil, ethyl vanillin and nonalactone gamma.

12. A method for preparing a flavouring preparation, a food product, an oral care product or a beverage which comprises:
a) identifying at least one flavouring which elicits a well-being effect by the method of any one of claims 1-8;
b) formulating said at least one flavouring thus identified into a flavouring preparation, a food product, an oral care product or a beverage.

13. Use a flavouring which elicits a well-being effect in a flavouring preparation, a food product, an oral care product or a beverage.

14. The use of claim 13, wherein the flavouring comprises one or more of orange oil, citrus oil, ethyl vanillin and nonalactone gamma.
